# EUROPEAN PATENT SPECIFICATION

(11) **EP 3 976 591 B1**
(45) Date of publication and mention of the grant of the patent: **19.04.2023**
(21) Application number: 20804381.0
(22) Date of filing: 16.10.2020
(51) Int. Cl.: C07D 231/16, C07D 401/04, C07D 405/04

(54) **METHODS FOR THE PREPARATION OF 5-BROMO-2-(3-CHLORO-PYRIDIN-2-YL)-2H-PYRAZOLE-3-CARBOXYLIC ACID**
VERFAHREN ZUR HERSTELLUNG VON 5-BROM-2-( 3-CHLOR-PYRIDIN-2-YL) -2H-PYRAZOL-3-CARBONSÄURE
PROCÉDÉS DE PRÉPARATION D'ACIDE 5-BROMO-2-(3CHLORO-PYRIDIN-2-YL)-2H-PYRAZOLE-3-CARBOXYLIQUE

(30) Priority: 18.10.2019 US 201962916840 P; 27.02.2020 US 202062982248 P
(43) Date of publication of application: 06.04.2022
(73) Proprietor: FMC Corporation, Philadelphia, Pennsylvania 19104 (US); FMC Agro Singapore Pte. Ltd., Singapore 018983 (SG)
(72) Inventor: CAO, Yanchun, Philadelphia, PA 19104 (US); LIU, Xin, Philadelphia, PA 19104 (US); MAO, Jianhua, Philadelphia, PA 19104 (US); XU, Zhijian, Philadelphia, PA 19104 (US)
(74) Representative: Dehns
(86) International application number: PCT/US2020/055888
(87) International publication number: WO 2021/076835

(56) References cited:
- EP-A1- 2 145 885
- EP-A1- 2 189 455
- EP-A1- 2 719 697
- WO-A1-2004/067528
- WO-A1-2006/068669
- WO-A1-2012/030922
- WO-A1-2017/059966
- CN-A- 105 669 643
- US-A1- 2015 291 572
- R. Miethchen R. Randow R. Listemann J. Hildebrandt K. Kohlheim: "Micellaktivierte lodierung und partielle Dehalogenierung von Pyrarolen und 1,2,4-Triazolen Micelle Activated Reactions I. Micelle Activated Iodination and Partial Dehalogenation of Pyrazoles and 1,2,4-Triazoles", Journal für Praktische Chemie, vol. 331, no. 5 1 January 1989 (1989-01-01), 1989, pages 799-805, XP055757253, Retrieved from the Internet: URL:https://doi.org/10.1002/prac.198933105 14

## Description

### FIELD OF INVENTION

This disclosure is directed to novel methods of synthesizing compounds including 5-bromo-2-(3-chloro-pyridin-2-yl)-2H-pyrazole-3-carboxylic acid. Compounds prepared by the methods disclosed herein are useful for preparation of certain anthranilamide compounds that are of interest as insecticides, such as, for example, the insecticides chlorantraniliprole and cyantraniliprole.

### BACKGROUND

Conventional processes for the production of 5-Bromo-2-(3-chloro-pyridin-2-yl)-2H-pyrazole-3-carboxylic acid are subject to several industrial concerns, such as processability, environmental hazards, high cost, reagent reactivity, and necessary specialized equipment.

The present disclosure provides novel methods useful for preparing 5-Bromo-2-(3-chloro-pyridin-2-yl)-2H-pyrazole-3-carboxylic acid and derivatives thereof. The benefits of the methods of the present disclosure compared to previous methods are numerous and include improved overall yield, reduced cost, eliminated need for mixed solvent separations, reduced waste, simplified operation complexity, and reduced process hazards.

The disclosed methods provide an overall yield of about 50% with commercially available and easily handled reagents.

EP 2145885 A1 discloses hydrazide compounds and methods for producing these compounds. WO 2006/068669 A1 discloses compositions comprising 3-bromo-N-[4-cyano-2-methyl-6-[(methylamino)carbonyl]phenyl]-1-(3-chloro-2-pyridinyl)-1H-pyrazole-5-carboxamide and methods for producing this compound. WO 2004/067528 A1 discloses anthranilamide insecticides and methods for producing these compounds.
CN 105669643 A discloses o-carboxamido benzamide derivatives and methods for preparing these compounds.

### BRIEF DESCRIPTION

In one aspect, provided herein is a method of preparing a compound of Formula VI, wherein 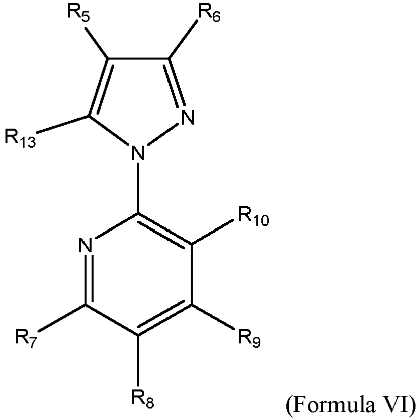
each of R₅ - R₁₀ is independently selected from hydrogen and halogen; and
R₁₃ is an organic acid, the method comprising
   I) forming a mixture comprising
      A) a compound of Formula V, wherein 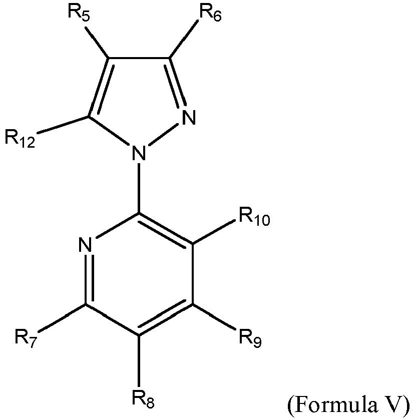
         each of R₅ - R₁₀ is independently selected from hydrogen and halogen;
         R₁₂ is an ester; and
         wherein the compound of Formula V is prepared according to a method comprising
            i) forming a mixture comprising
               a) a compound of Formula III, wherein 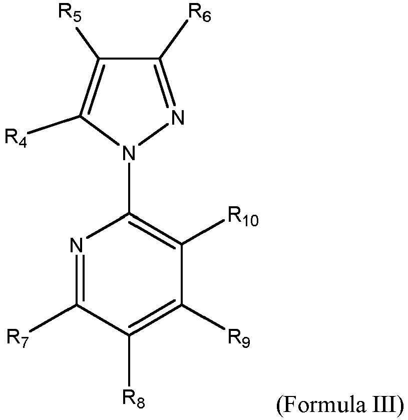 each of R₄ - R₁₀ is independently selected from hydrogen and halogen; and wherein at least one of R₄, R₅, and R₆ is hydrogen;
               b) a first solvent;
               c) a second solvent;
               d) a compound comprising a metal; and
               e) optionally an additive; and
            ii) reacting the mixture; and
      B) a metal hydroxide; and
II) reacting the mixture.

In one aspect, provided herein is a method of preparing a compound of Formula V, wherein 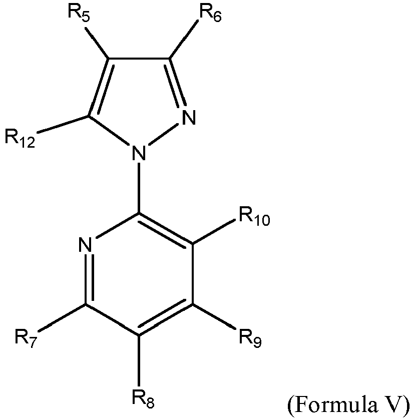
each of R₅ - R₁₀ is independently selected from hydrogen and halogen; and
R₁₂ is an ester, the method comprising
   I) forming a mixture comprising
      A) a compound of Formula III, wherein 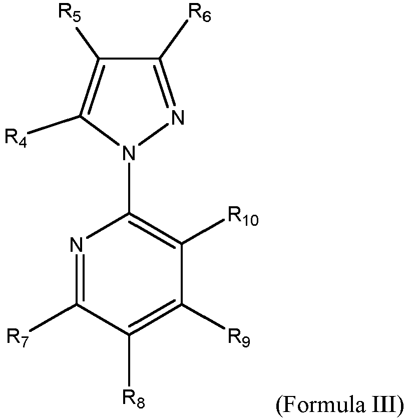
         R₄ is a halogen; and
         each of R₅ - R₁₀ is independently selected from hydrogen and halogen;
      B) a first solvent;
      C) a second solvent;
      D) a compound comprising a metal; and
      E) optionally an additive; and
   II) reacting the mixture.

The compound of Formula III used in the invention, 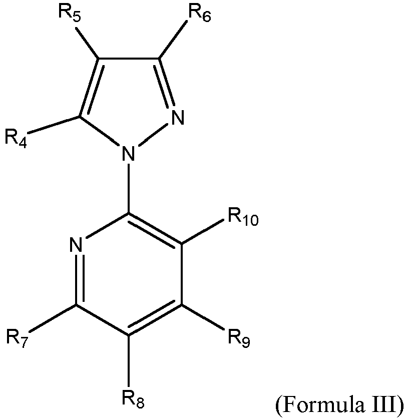
wherein each of R₄ - R₁₀ is independently selected from hydrogen and halogen; and
wherein at least one of R₄, R₅, and R₆ is hydrogen, may be prepared by a method comprising
   I) forming a mixture comprising
      A) a compound of Formula II, wherein 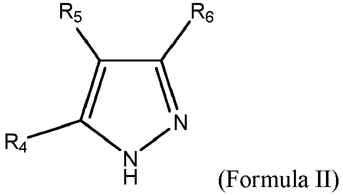
         each of R₄, R₅, and R₆ is independently selected from hydrogen and halogen;
         wherein at least one of R₄, R₅, and R₆ is hydrogen; and
         wherein the compound of Formula II is prepared according to a method comprising
            i) forming a mixture comprising
               a) a compound of Formula I, wherein 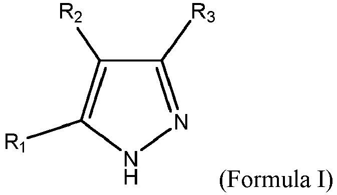 each of R₁, R₂, and R₃ is independently a halogen;
               b) optionally a dehalogenation reagent;
               c) a reducing agent; and
               d) a solvent; and
            ii) reacting the mixture;
      B) a compound of Formula IV, wherein 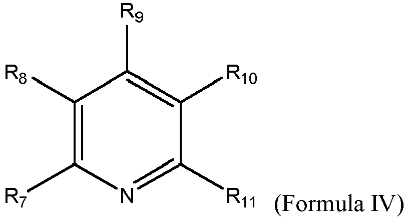 each of R₇ - R₁₁ is independently selected from hydrogen and halogen;
      C) a solvent;
      D) an inorganic base; and
      E) optionally an additive; and
   II) reacting the mixture.

A compound of Formula II 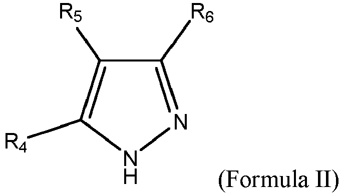
wherein each of R₄, R₅, and R₆ is independently selected from hydrogen and halogen; and
wherein at least one of R₄, R₅, and R₆ is hydrogen, can be prepared by a method comprising
   I) forming a mixture comprising
      A) a compound of Formula I, wherein 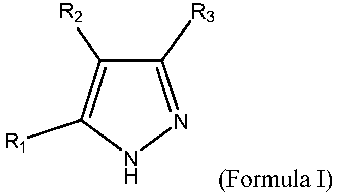
         each of R₁, R₂, and R₃ is independently a halogen; and
         wherein the compound of Formula I is prepared according to a method comprising
            i) forming a mixture comprising
               a) pyrazole or a pyrazole derivative;
               b) a halogenation reagent;
               c) water;
               d) optionally a solvent; and
               e) optionally an inorganic base; and
            ii) reacting the mixture;
      B) optionally a dehalogenation reagent;
      C) a reducing agent; and
      D) a solvent; and
   II) reacting the mixture.

A compound of Formula II-A 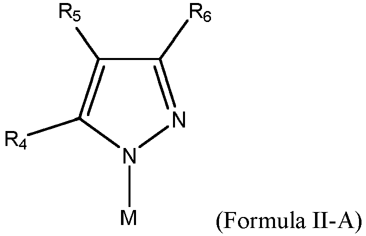
wherein M is selected from alkali metals and alkaline metals;
each of R₄, R₅, and R₆ is independently selected from hydrogen and halogen; and
wherein at least one of R₄, R₅, and R₆ is hydrogen, can be prepared by a method comprising
   I) forming a mixture comprising
      A) a compound of Formula I, wherein 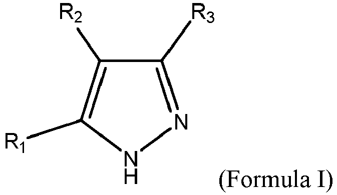
         each of R₁, R₂, and R₃ is independently a halogen; and
         wherein the compound of Formula I is prepared according to a method comprising
            i) forming a mixture comprising
               a) pyrazole or a pyrazole derivative;
               b) a halogenation reagent;
               c) water;
               d) optionally a solvent; and
               e) optionally an inorganic base; and
            ii) reacting the mixture;
      B) optionally a dehalogenation reagent;
      C) a reducing agent; and
      D) a solvent; and
   II) reacting the mixture.

Compounds of Formula II-A as disclosed herein may have the structure 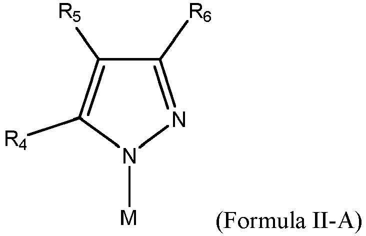 wherein:
M is selected from alkali metals and alkaline metals;
each of R₄, R₅, and R₆ is independently selected from hydrogen and halogen; and
wherein at least one of R₄, R₅, and R₆ is hydrogen.

### DETAILED DESCRIPTION OF THE DISCLOSURE

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process or method.

The transitional phrase "consisting of" excludes any element, step, or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of" is used to define a composition or method that includes materials, steps, features, components, or elements, in addition to those literally disclosed, provided that these additional materials, steps, features, components, or elements do not materially affect the basic and novel characteristic(s) of the claimed invention. The term "consisting essentially of" occupies a middle ground between "comprising" and "consisting of".

Where an invention or a portion thereof is defined with an openended term such as "comprising," it should be readily understood that (unless otherwise stated) the description should be interpreted to also describe such an invention using the terms "consisting essentially of" or "consisting of."

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

As used herein, the term "about" means plus or minus 10% of the value.

The term "halogen", either alone or in compound words such as "haloalkyl", includes fluorine, chlorine, bromine or iodine. Further, when used in compound words such as "haloalkyl", said alkyl may be partially or fully substituted with halogen atoms which may be the same or different.

When a group contains a substituent which can be hydrogen, for example R⁴, then, when this substituent is taken as hydrogen, it is recognized that this is equivalent to said group being unsubstituted.

The term "organic base" includes, without limitation, amine compounds (e.g., primary, secondary and tertiary amines), heterocycles including nitrogen-containing heterocycles, and ammonium hydroxide.

The term "inorganic base" includes, without limitation, inorganic compounds with the ability to react with, or neutralize, acids to form salts, such as, for example, metal salts of hydroxide, carbonate, bicarbonate and phosphate.

The term "halogenation reagent" includes, without limitation, halogens and inorganic compounds, such as, for example, bromine, NBS, and 1,3-dibromo-5,5-dimethylhylhydantoin.

The term "phase transfer catalyst" includes compounds that facilitate the migration of a reactant from one phase into another phase where a reaction occurs. Phase transfer catalysis refers to the acceleration of the reaction upon the addition of the phase transfer catalyst.

The term "ester" includes, without limitation, a functional group comprising an ester bond (C(=O)-O-). In some aspects, the functional group comprising an ester bond is an alkyl (or cycloalkyl) having one to eight carbon atoms, like methyl, ethyl, 1 -propyl, 2-propyl, 1 - butyl, 1-methylheptyl (meptyl), etc.

The term "ether" includes, without limitation, a functional group comprising an ether bond (C-O-C).

The term "nitrile" includes, without limitation, a functional group comprising a nitrile bond (-C≡N).

The term "carboxylic acid" includes, without limitation, a functional group comprising a carboxylic acid bond (C(=O)-OH).

The term "organic acid" includes, without limitation, a functional group that confers acidity and consists of atoms selected from carbon, nitrogen, oxygen, and hydrogen.

Certain compounds used in this invention can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers.

The embodiments of this disclosure include:
Embodiment 1. A method of preparing a compound of Formula VI, wherein 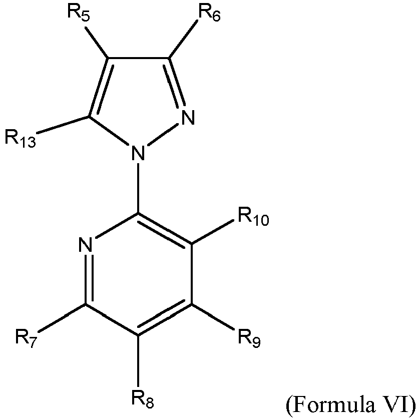
   each of R₅ - R₁₀ is independently selected from hydrogen and halogen; and
   R₁₃ is an organic acid, the method comprising
      I) forming a mixture comprising
         A) a compound of Formula V, wherein 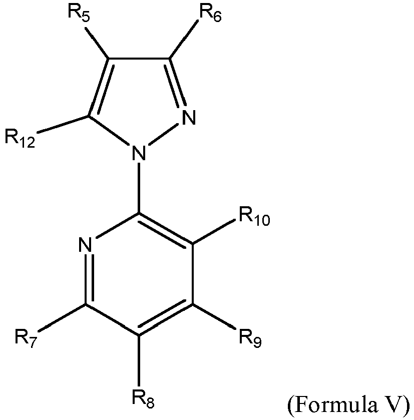
            each of R₅ - R₁₀ is independently selected from hydrogen and halogen;
            R₁₂ is an ester; and
            wherein the compound of Formula V is prepared according to a method comprising
               i) forming a mixture comprising
                  a) a compound of Formula III, wherein 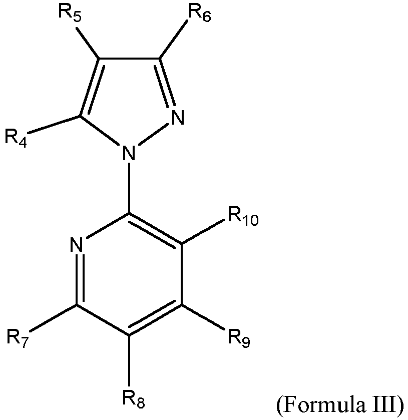 each of R₄ - R₁₀ is independently selected from hydrogen and halogen; and wherein at least one of R₄, R₅, and R₆ is hydrogen;
                  b) a first solvent;
                  c) a second solvent;
                  d) a compound comprising a metal; and
                  e) optionally an additive; and
               ii) reacting the mixture; and
         B) a metal hydroxide; and
      II) reacting the mixture.
Embodiment 2. The method of embodiment 1, wherein the metal hydroxide is selected from alkali hydroxide, alkaline earth metal hydroxide, and combinations thereof.
Embodiment 3. The method of embodiment 2, wherein the alkali hydroxide is selected from lithium hydroxide, sodium hydroxide, and potassium hydroxide.
Embodiment 4. The method of embodiment 2, wherein the alkaline earth metal hydroxide is selected from calcium hydroxide and barium hydroxide.
Embodiment 5. The method of embodiment 1, wherein the method step II) of reacting the mixture occurs at a reaction temperature in the range of about 0 °C to about 90 °C.
Embodiment 6. The method of embodiment 1, wherein the compound comprising a metal is selected from a Grignard reagent, and a lithium-containing compound.
Embodiment 7. The method of embodiment 6, wherein the Grignard reagent is selected from MeMgCl, iPrMgCl, iPrMgBr, EtMgCl, and combinations thereof.
Embodiment 8. The method of embodiment 7, wherein the Grignard reagent is iPrMgBr.
Embodiment 9. The method of embodiment 6, wherein the lithium-containing compound is nBuLi.
Embodiment 10. The method of embodiment 1, wherein the first solvent is selected from THF, toluene, 1,4-dioxane, Me-THF, and combinations thereof.
Embodiment 11. The method of embodiment 10, wherein the first solvent is THF.
Embodiment 12. The method of embodiment 1, wherein the second solvent is selected from dimethyl carbonate, N,N-dimethyacetamide, and combinations thereof.
Embodiment 13. The method of embodiment 12, wherein the second solvent is dimethyl carbonate.
Embodiment 14. The method of embodiment 1, wherein the method step ii) of reacting the mixture occurs at a reaction temperature in the range of about 0 °C to about 60 °C.
Embodiment 15. The method of embodiment 14, wherein the method step ii) of reacting the mixture occurs at a reaction temperature in the range of about 0 °C to about 30 °C.
Embodiment 16. The method of embodiment 1, wherein R₄, R₅, and R₆ of Formula III are each independently hydrogen.
Embodiment 17. The method of embodiment 1, wherein the compound of Formula III is prepared according to a method comprising
   I) forming a mixture comprising
      A) a compound of Formula II, wherein 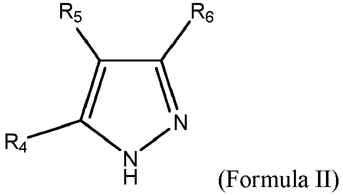
         each of R₄, R₅, and R₆ is independently selected from hydrogen and halogen; and
         wherein at least one of R₄, R₅, and R₆ is hydrogen;
      B) a compound of Formula IV, wherein 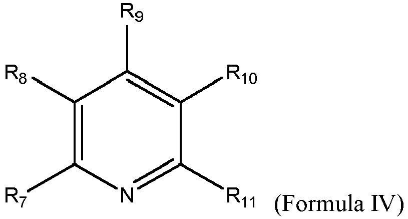 each of R₇ - R₁₁ is independently selected from hydrogen and halogen;
      C) a solvent;
      D) an inorganic base; and
      E) optionally an additive; and
   II) reacting the mixture.
Embodiment 18. The method of embodiment 17, wherein the inorganic base is selected from powder sodium hydroxide, powder potassium hydroxide, potassium carbonate, sodium carbonate, potassium phosphate, powder sodium methoxide, powder potassium t-butoxide, and combinations thereof.
Embodiment 19. The method of embodiment 17, wherein the solvent is selected from toluene, N,N-dimethylformamide, N,N-dimethylacetamide, N-methyl-2-pyrrolidone, NMP, sulfolane, triglyme, diglyme and combinations thereof.
Embodiment 20. The method of embodiment 17, wherein the additive is selected from potassium iodide, a phase transfer catalyst, and combinations thereof.
Embodiment 21. The method of embodiment 17, wherein the phase transfer catalyst is selected from butyl ammonium chloride, tetra butyl ammonium bromide, aliquat-336, 18-crown-6, and combinations thereof.
Embodiment 22. The method of embodiment 17, wherein the method step II) of reacting the mixture occurs at a reaction temperature in the range of about 140 °C to about 200 °C.
Embodiment 23. The method of embodiment 17, wherein the compound of Formula II is prepared according to a method comprising
   I) forming a mixture comprising
      A) a compound of Formula I, wherein 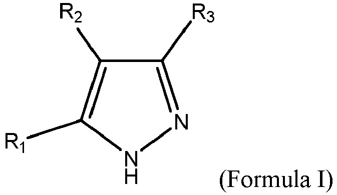 each of R₁, R₂, and R₃ is independently a halogen;
      B) optionally a dehalogenation reagent;
      C) a reducing agent; and
      D) a solvent; and
   II) reacting the mixture.
Embodiment 24. The method of embodiment 23, wherein the solvent is selected from acetic acid, water, toluene, p-dichlorobenzene, N,N-dimethylformamide, N,N-dimethylacetamide, sulfolane, N-methyl-2-pyrrolidone (NMP), and combinations thereof.
Embodiment 25. The method of embodiment 23, wherein the reducing agent is selected from sodium sulfite, sodium bisulfite, sodium hyposulfite, sodium thiosulfate, sodium hydrosulfide, sodium sulfate, and combinations thereof.
Embodiment 26. The method of embodiment 23, wherein the dehalogenation reagent is selected from sodium iodide, iodine, potassium iodide, tetra-n-butyl ammonium iodide, and combinations thereof.
Embodiment 27. The method of embodiment 23, wherein the method step II) of reacting the mixture occurs at a reaction temperature in the range of about 100 °C to about 180 °C.
Embodiment 28. The method of embodiment 23, wherein the compound of Formula I is prepared according to a method comprising
   I) forming a mixture comprising
      A) pyrazole or a pyrazole derivative;
      B) a halogenation reagent;
      C) water; and
      D) optionally a solvent; and
      E) optionally an inorganic base; and
   II) reacting the mixture.
Embodiment 29. The method of embodiment 28, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, and combinations thereof; and
   B) optionally hydrogen peroxide.
Embodiment 30. The method of embodiment 28, wherein the inorganic base is selected from powder sodium hydroxide, sodium hydroxide solution, powder sodium acetate, and combinations thereof.
Embodiment 31. The method of embodiment 28, wherein the method step II) of reacting the mixture occurs at a reaction temperature in the range of about 0 °C to about 70 °C.
Embodiment 32. A method of preparing a compound of Formula V, wherein 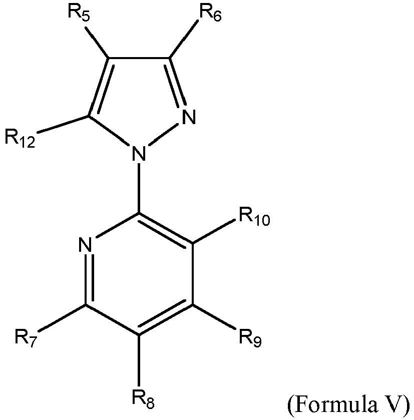
   each of R₅ - R₁₀ is independently selected from hydrogen and halogen; and
   R₁₂ is an ester, the method comprising
      I) forming a mixture comprising
         A) a compound of Formula III, wherein 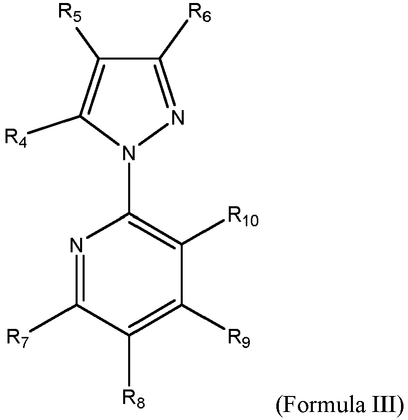
            R₄ is a halogen; and
            each of R₅ - R₁₀ is independently selected from hydrogen and halogen;
         B) a first solvent;
         C) a second solvent;
         D) a compound comprising a metal; and
         E) optionally an additive; and
      II) reacting the mixture.
Embodiment 33. The method of embodiment 32, wherein the compound comprising a metal is selected from a Grignard reagent and a lithium-containing compound.
Embodiment 34. The method of embodiment 33, wherein the Grignard reagent is selected from MeMgCl, iPrMgCl, iPrMgBr, EtMgCl, and combinations thereof.
Embodiment 35. The method of embodiment 34, wherein the Grignard reagent is iPrMgBr.
Embodiment 36. The method of embodiment 33, wherein the lithium-containing compound is nBuLi.
Embodiment 37. The method of embodiment 32, wherein the first solvent is selected from THF, toluene, 1,4-dioxane, Me-THF, and combinations thereof.
Embodiment 38. The method of embodiment 37, wherein the first solvent is THF.
Embodiment 39. The method of embodiment 32, wherein the second solvent is selected from dimethyl carbonate, N,N-dimethyacetamide, and combinations thereof.
Embodiment 40. The method of embodiment 39, wherein the second solvent is dimethyl carbonate.
Embodiment 41. The method of embodiment 32, wherein the method step II) of reacting the mixture occurs at a reaction temperature in the range of about 0 °C to about 60 °C.
Embodiment 42. The method of embodiment 41, wherein the method step II) of reacting the mixture occurs at a reaction temperature in the range of about 0 °C to about 30 °C.
Embodiment 43. The method of embodiment 32, wherein R₄, R₅, and R₆ of Formula III are each independently hydrogen.
The compound of Formula (III) used in the methods of the invention may be prepared according to a method as defined in Embodiments 46-59.
Embodiment 46. A method of preparing a compound of Formula III, wherein 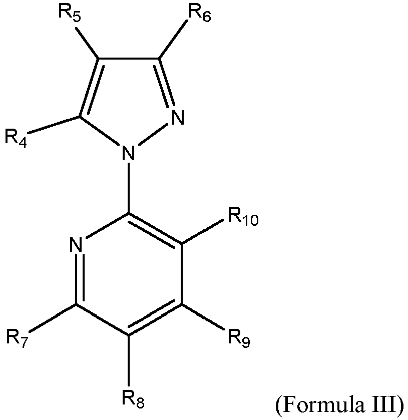
   each of R₄ - R₁₀ is independently selected from hydrogen and halogen; and
   wherein at least one of R₄, R₅, and R₆ is hydrogen, the method comprising
      I) forming a mixture comprising
         A) a compound of Formula II, wherein 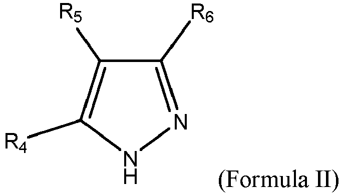
            each of R₄, R₅, and R₆ is independently selected from hydrogen and halogen;
            wherein at least one of R₄, R₅, and R₆ is hydrogen; and
            wherein the compound of Formula II is prepared according to a method comprising
               i) forming a mixture comprising
                  a) a compound of Formula I, wherein 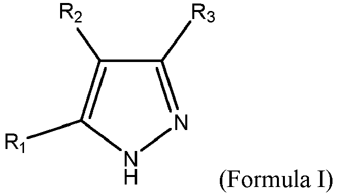 each of R₁, R₂, and R₃ is independently a halogen;
                  b) optionally a dehalogenation reagent;
                  c) a reducing agent; and
                  d) a solvent; and
               ii) reacting the mixture;
         B) a compound of Formula IV, wherein 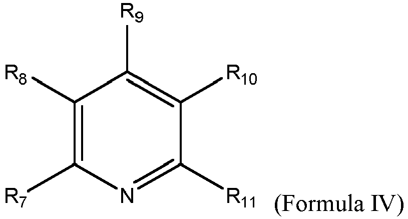 each of R₇ - R₁₁ is independently selected from hydrogen and halogen;
         C) a solvent;
         D) an inorganic base; and
         E) optionally an additive; and
      II) reacting the mixture.
Embodiment 47. The method of embodiment 46, wherein the inorganic base is selected from powder sodium hydroxide, powder potassium hydroxide, potassium carbonate, sodium carbonate, potassium phosphate, powder sodium methoxide, powder potassium t-butoxide, and combinations thereof.
Embodiment 48. The method of embodiment 46, wherein the solvent C) is selected from toluene, N,N-dimethylformamide, N,N-dimethylacetamide, N-methyl-2-pyrrolidone, NMP, sulfolane, diglyme, triglyme and combinations thereof.
Embodiment 49. The method of embodiment 46, wherein the additive is selected from potassium iodide, a phase transfer catalyst, and combinations thereof.
Embodiment 50. The method of embodiment 49, wherein the phase transfer catalyst is selected from butyl ammonium chloride, tetra butyl ammonium bromide, aliquat-336, 18-crown-6, and combinations thereof.
Embodiment 51. The method of embodiment 46, wherein the method step II) of reacting the mixture occurs at a reaction temperature in the range of about 140 °C to about 200 °C.
Embodiment 52. The method of embodiment 46, wherein the solvent d) is selected from acetic acid, water, toluene, N,N-dimethylformamide, N,N-dimethylacetamide, and combinations thereof.
Embodiment 53. The method of embodiment 46, wherein the reducing agent is selected from sodium sulfite, sodium bisulfite, sodium hyposulfite, sodium thiosulfate, sodium hydrosulfide, sodium sulfate, and combinations thereof.
Embodiment 54. The method of embodiment 46, wherein the dehalogenation reagent is selected from sodium iodide, iodine, potassium iodide, tetra-n-butyl ammonium iodide, and combinations thereof.
Embodiment 55. The method of embodiment 46, wherein the method step ii) of reacting the mixture occurs at a reaction temperature in the range of about 100 °C to about 180 °C.
Embodiment 56. The method of embodiment 46, wherein the compound of Formula I is prepared according to a method comprising
   I) forming a mixture comprising
      A) pyrazole or a pyrazole derivative;
      B) a halogenation reagent;
      C) water; and
      D) optionally a solvent; and
      E) optionally an inorganic base; and
   II) reacting the mixture.
Embodiment 57. The method of embodiment 56, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, and combinations thereof; and
   B) optionally hydrogen peroxide.
Embodiment 58. The method of embodiment 56, wherein the inorganic base is selected from powder sodium hydroxide, sodium hydroxide solution, powder sodium acetate, and combinations thereof.
Embodiment 59. The method of embodiment 56, wherein the method step II) of reacting the mixture occurs at a reaction temperature in the range of about 0 °C to about 70 °C.

A compound of Formula II may be prepared according to a method as defined in Embodiments 60-67.

Embodiment 60. A method of preparing a compound of Formula II, wherein 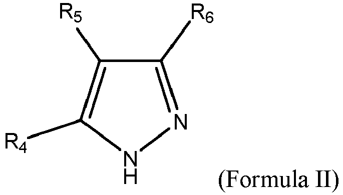
each of R₄, R₅, and R₆ is independently selected from hydrogen and halogen; and
wherein at least one of R₄, R₅, and R₆ is hydrogen, the method comprising
   I) forming a mixture comprising
      A) a compound of Formula I, wherein 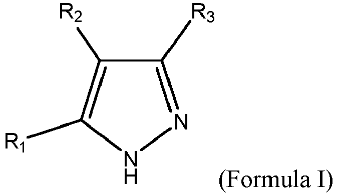
         each of R₁, R₂, and R₃ is independently a halogen; and
         wherein the compound of Formula I is prepared according to a method comprising
            i) forming a mixture comprising
               a) pyrazole or a pyrazole derivative;
               b) a halogenation reagent;
               c) water; and
               d) optionally a solvent; and
               e) optionally an inorganic base; and
            ii) reacting the mixture;
      B) optionally a dehalogenation reagent;
      C) a reducing agent; and
      D) a solvent; and
   II) reacting the mixture.

Embodiment 61-1. The method of embodiment 60, wherein the solvent in d) is selected from alkanes, ethers, halogenated solvents, esters, water, and combinations thereof. In some embodiments, the halogenated solvent is selected from dichloromethane, methylene chloride, trichloroethylene, and combinations thereof. In some embodiments, the ester solvent is selected from butyl acetate, sec-butyl acetate, tert-butyl acetate, and combinations thereof. In some embodiments, the solvent is an ether solvent selected from methyl tert-butyl ether (MTBE), diethyl ether (Et2O), and combinations thereof.

Embodiment 61-11. The method of embodiment 60, wherein the solvent D) is selected from acetic acid, water, toluene, N,N-dimethylformamide, N,N-dimethylacetamide, and combinations thereof.

Embodiment 62. The method of embodiment 60, wherein the reducing agent is selected from sodium sulfite, sodium bisulfite, sodium hyposulfite, sodium thiosulfate, sodium hydrosulfide, sodium sulfate, and combinations thereof.

Embodiment 63. The method of embodiment 60, wherein the dehalogenation reagent is selected from sodium iodide, iodine, potassium iodide, tetra-n-butyl ammonium iodide, and combinations thereof.

Embodiment 64. The method of embodiment 60, wherein the method step II) of reacting the mixture occurs at a reaction temperature in the range of about 100 °C to about 180 °C.

Embodiment 65. The method of embodiment 60, wherein the halogenation reagent comprises
A) a reagent selected from hydrogen bromide, bromine, N-bromosuccinimide, 1,3-dibromo-5,5-dimethylhylhydantoin, sodium bromide, potassium bromide, and combinations thereof; and
B) optionally hydrogen peroxide.

Embodiment 66. The method of embodiment 60, wherein the inorganic base is selected from powder sodium hydroxide, sodium hydroxide solution, powder sodium acetate, and combinations thereof.

Embodiment 67. The method of embodiment 60, wherein the method step ii) of reacting the mixture occurs at a reaction temperature in the range of about 0 °C to about 70 °C.

A compound of Formula II-A may be prepared by a method as defined in Embodiments 110-119.

Embodiment 110. A method of preparing a compound of Formula II-A, wherein 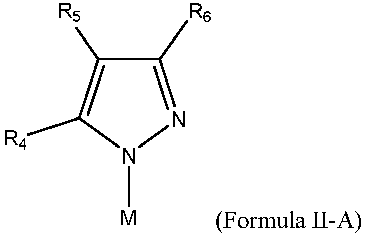
M is selected from alkali metals and alkaline metals;
each of R₄, R₅, and R₆ is independently selected from hydrogen and halogen; and
wherein at least one of R₄, R₅, and R₆ is hydrogen, the method comprising
   I) forming a mixture comprising
      A) a compound of Formula I, wherein 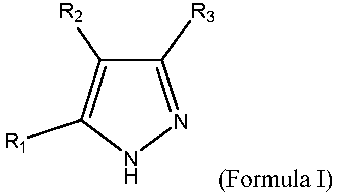
         each of R₁, R₂, and R₃ is independently a halogen; and
         wherein the compound of Formula I is prepared according to a method comprising
            i) forming a mixture comprising
               a) pyrazole or a pyrazole derivative;
               b) a halogenation reagent;
               c) water; and
               d) optionally an inorganic base; and
            ii) reacting the mixture;
      B) optionally a dehalogenation reagent;
      C) a reducing agent; and
      D) a solvent; and
   II) reacting the mixture.

Embodiment 111-I. The method of embodiment 60, wherein the solvent in d) is selected from alkanes, ethers, halogenated solvents, esters, water, and combinations thereof. In some embodiments, the halogenated solvent is selected from dichloromethane, methylene chloride, trichloroethylene, and combinations thereof. In some embodiments, the ester solvent is selected from butyl acetate, sec-butyl acetate, tert-butyl acetate, and combinations thereof. In some embodiments, the solvent is an ether solvent selected from methyl tert-butyl ether (MTBE), diethyl ether (Et2O), and combinations thereof.

Embodiment 111-II. The method of embodiment 110, wherein the solvent D) is selected from acetic acid, water, toluene, N,N-dimethylformamide, N,N-dimethylacetamide, and combinations thereof.

Embodiment 112. The method of embodiment 110, wherein the reducing agent is selected from sodium sulfite, sodium bisulfite, sodium hyposulfite, sodium thiosulfate, sodium hydrosulfide, sodium sulfate, and combinations thereof.

Embodiment 113. The method of embodiment 110, wherein the dehalogenation reagent is selected from sodium iodide, iodine, potassium iodide, tetra-n-butyl ammonium iodide, and combinations thereof.

Embodiment 114. The method of embodiment 110, wherein the method step II) of reacting the mixture occurs at a reaction temperature in the range of about 100 °C to about 180 °C.

Embodiment 115. The method of embodiment 110, wherein the halogenation reagent comprises
A) a reagent selected from hydrogen bromide, bromine, N-bromosuccinimide, 1,3-dibromo-5,5-dimethylhylhydantoin, sodium bromide, potassium bromide, and combinations thereof; and
B) optionally hydrogen peroxide.

Embodiment 116. The method of embodiment 110, wherein the inorganic base is selected from powder sodium hydroxide, sodium hydroxide solution, powder sodium acetate, and combinations thereof.

Embodiment 117. The method of embodiment 110, wherein the method step ii) of reacting the mixture occurs at a reaction temperature in the range of about 0 °C to about 70 °C.

Embodiment 118. The method of embodiment 110, wherein M is selected from lithium, sodium, potassium, calcium, and magnesium.

Embodiment 119. The method of embodiment 110, wherein the compound of Formula II-A is

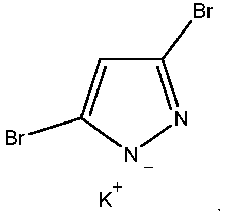

Compounds of Formula II-A as disclosed herein may have the following structure 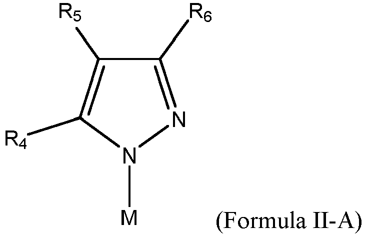 wherein
M is selected from alkali metals and alkaline metals;
each of R₄, R₅, and R₆ is independently selected from hydrogen and halogen; and
wherein at least one of R₄, R₅, and R₆ is hydrogen.

An example of a compound of Formula II-A is

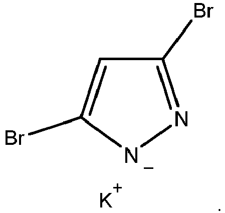

In one aspect, a compound of Formula VI is prepared according to a method represented by Scheme 1. The R groups are as defined anywhere in this disclosure.

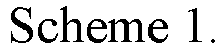

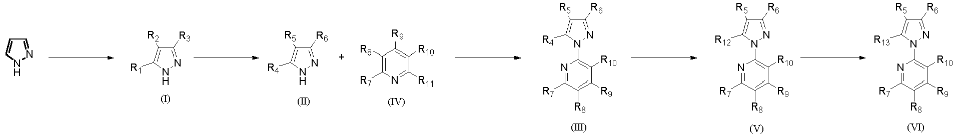

In one aspect, 5-Bromo-2-(3-chloro-pyridin-2-yl)-2H-pyrazole-3-carboxylic acid is prepared according to a method represented by Scheme 2.

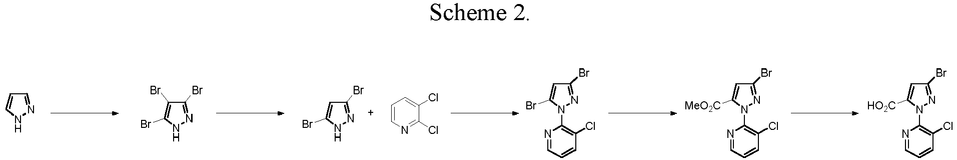

A compound of Formula I may be prepared according to a method represented by Scheme 4. The R groups are as defined anywhere in this disclosure.

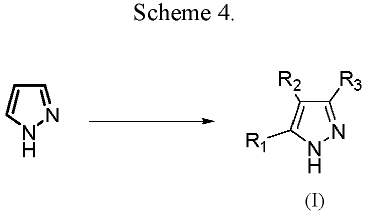

This may include reacting pyrazole with a halogenation reagent in water, and optionally a solvent and further optionally in the presence of an inorganic base. In one embodiment, the halogenation reagent is selected from hydrogen peroxide/HBr, Bromine (Br₂), N-bromosuccinimide, 1,3-dibromo-5,5-dimethylhylhydantoin, hydrogen peroxide/NaBr, hydrogen peroxide/KBr, hydrogen peroxide/Br₂, and combinations thereof. In another embodiment, the halogenation reagent is hydrogen peroxide/HBr. In one embodiment, inorganic base is selected from powder sodium hydroxide, sodium hydroxide solution, powder sodium acetate, and combinations thereof. In another embodiment there is no base. In one embodiment, the reaction temperature is in the range from about 0 °C to about 70 °C. In another embodiment, the reaction temperature is in the range from about 0 °C to about 30 °C. In yet another embodiment, the solvent is selected from alkanes, ethers, halogenated solvents, esters, water, and combinations thereof. In some embodiments, the solvent is an ether solvent selected from methyl tert-butyl ether (MTBE), diethyl ether (Et2O), and combinations thereof.

A compound of Formula II may be prepared according to a method represented by Scheme 5. The R groups are as defined anywhere in this disclosure.

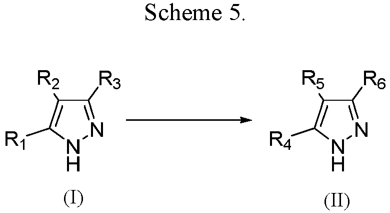

This method may include reacting a compound of Formula I with a dehalogenation reagent in a solvent in the presence of a reducing agent. In one embodiment, the solvent is selected form aromatic solvents, halogenated aromatic solvents, and combinations thereof. In one embodiment, the solvent is selected from acetic acid, water, toluene, N,N-dimethylformamide (DMF), N,N-dimethylacetamide (DMAc), N-methyl-2-pyrrolidone (NMP), sulfolane and combinations thereof. In another embodiment, the solvent is N,N-dimethylacetamide (DMAc). In yet another embodiment, the solvent is sulfolane. In one embodiment, the dehalogenation reagent is selected from sodium iodide, iodine, potassium iodide, tetra-n-butyl ammonium iodide (TBAI), and combinations thereof. In another embodiment, the dehalogenation reagent is potassium iodide. In one embodiment, the reducing agent is selected from sodium sulfite, sodium bisulfite, sodium hyposulfite, sodium thiosulfate, sodium hydrosulfide, sodium sulphate, and combinations thereof. In another embodiment, the reducing agent is sodium sulfite. In one embodiment, the reaction temperature is in the range from about 100 °C to about 180 °C. In another embodiment, the reaction temperature is in the range from about 160 °C to about 180 °C.

A compound of Formula II-A may be prepared according to a method represented by Scheme 6. The R groups are as defined anywhere in this disclosure.

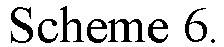

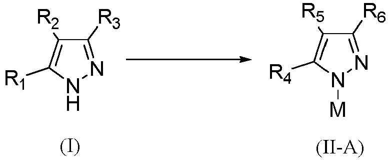

The compound of Formula II-A may be a metal salt of Formula II. The bond between M and N may be an ionic bond. M may be selected from alkali metals and alkaline metals. M may be selected from lithium, sodium, and potassium, such as M is potassium. Alternatively, M may be selected from calcium and magnesium.

One example of a compound of Formula II-A is

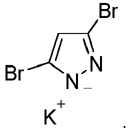

This method may include reacting a compound of Formula I with a dehalogenation reagent in a solvent in the presence of a reducing agent. The solvent may be selected form aromatic solvents, halogenated aromatic solvents, and combinations thereof. The solvent may be selected from acetic acid, water, toluene, N,N-dimethylformamide (DMF), N,N-dimethylacetamide (DMAc), N-methyl-2-pyrrolidone (NMP), sulfolane and combinations thereof, such as N,N-dimethylacetamide (DMAc). The solvent may be sulfolane. The dehalogenation reagent may be selected from sodium iodide, iodine, potassium iodide, tetra-n-butyl ammonium iodide (TBAI), and combinations thereof, such as potassium iodide. The reducing agent may be selected from sodium sulfite, sodium bisulfite, sodium hyposulfite, sodium thiosulfate, sodium hydrosulfide, sodium sulphate, and combinations thereof, such as sodium sulfite. In one embodiment, the reaction temperature is in the range from about 100 °C to about 180 °C, such as from about 160 °C to about 180 °C.

A compound of Formula III may be prepared according to a method represented by Scheme 7. The R groups are as defined anywhere in this disclosure.

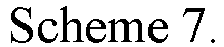

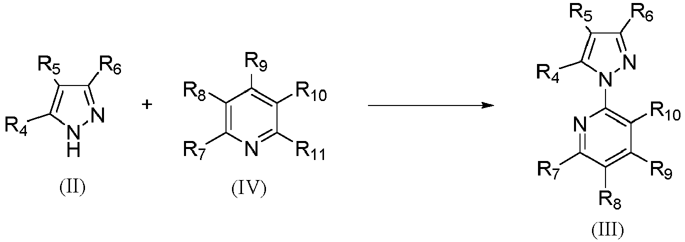

This method may include the step of mixing a compound of Formula II with a compound of Formula IV in a solvent in the presence of an inorganic base and optionally an additive. In one embodiment, the inorganic base is selected from powder sodium hydroxide, powder potassium hydroxide, sodium carbonate, potassium carbonate, potassium phosphate powder sodium methoxide, powder potassium t-butoxide, and combinations thereof. In one embodiment, the inorganic base is sodium carbonate. In one embodiment, the solvent is selected from toluene, N,N-dimethylformamide (DMF), N,N-dimethylacetamide (DMAc), N-methyl-2-pyrrolidone (NMP), sulfolane, diglyme, triglyme and combinations thereof. In another embodiment, the solvent is N,N-dimethyl-acetamide (DMAc). In yet another embodiment, the solvent is sulfolane. In one embodiment, the additive is a phase catalyst selected from butyl ammonium chloride (TBAC), tetra butyl ammonium bromide (TBAB), aliquat-336, 18-crown-6, and combinations thereof. In another embodiment, the phase catalyst is 18-crown-6. In another embodiment, the additive is potassium iodide. In one embodiment, the reaction temperature ranging is in the range from about 140 °C to about 200 °C. In another embodiment, the temperature is in the range from about 155 °C to about 175 °C.

In one aspect, a compound of Formula V is prepared according to a method represented by Scheme 8. The R groups are as defined anywhere in this disclosure.

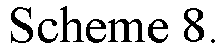

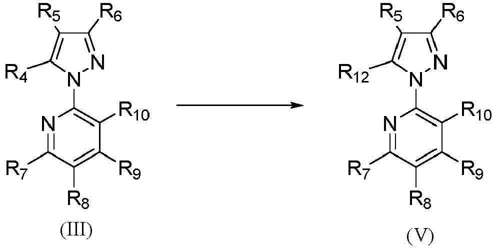

This aspect includes mixing a compound of Formula III with dimethyl carbonate (DMC) in a solvent in the presence of a base reagent and optionally an additive. In one embodiment, the base reagent is selected from MeMgCl, iPrMgCl, iPrMgBr, EtMgCl, nBuLi, and combinations thereof. In another embodiment, the base reagent is iPrMgBr. In one embodiment, the solvent is selected from tetrahydrofuran (THF), toluene, 1,4-dioxane, 2-methyltetrahydrofuran (Me-THF), and combinations thereof. In another embodiment, the solvent is THF. In one embodiment, the reaction temperature is in the range from about 0 °C to about 60 °C. In another embodiment, the temperature is in the range from about 0 °C to about 30°C.

In one aspect, a compound of Formula VI is prepared according to a method represented by Scheme 10. The R groups are as defined anywhere in this disclosure.

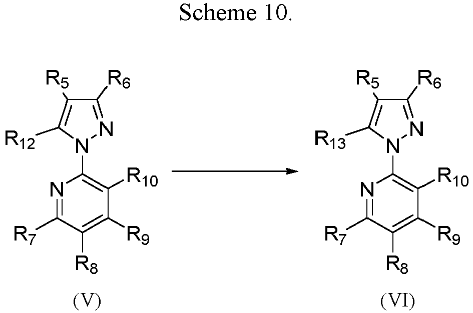

This aspect includes reacting a compound of Formula V with an aqueous metal hydroxide solution. In one embodiment, the metal hydroxide is selected from alkali hydroxide, alkaline earth metal hydroxide, and combinations thereof. In one embodiment, the alkali hydroxide is selected from lithium hydroxide, sodium hydroxide, potassium hydroxide, and combinations. In one embodiment, the alkaline earth metal hydroxide is selected from calcium hydroxide, barium hydroxide, and combinations thereof. In another embodiment, the metal hydroxide is sodium hydroxide or potassium hydroxide. In one embodiment, the reaction temperature is in the range from about 0 °C to about 90 °C. In another embodiment, the reaction temperature is in the range from about 60 °C to about 80 °C.

### EXAMPLES

Without further elaboration, it is believed that one skilled in the art using the preceding description can utilize the present invention to its fullest extent. The following Examples are, therefore, to be construed as merely illustrative, and not limiting of the disclosure in any way whatsoever. The starting material for the following Examples may not have necessarily been prepared by a particular preparative run whose procedure is described in other Examples. It also is understood that any numerical range recited herein includes all values from the lower value to the upper value. For example, if a range is stated as 10-50, it is intended that values such as 12-30, 20-40, or 30-50, etc., are expressly enumerated in this specification. These are only examples of what is specifically intended, and all possible combinations of numerical values between and including the lowest value and the highest value enumerated are to be considered to be expressly stated in this application.

Example 1. Hydrogen peroxide/HBr as a halogenation reagent.

34 grams of pyrazole and 505.8 g of 48% hydrogen bromide solution were charged to a reactor. 170 grams of 30% hydrogen peroxide was added drop-wise at 0 °C over 2 hours. The reaction temperature was controlled at 0-30 °C. After reaction, the product was precipitated as a solid, and then the reaction mixture was quenched with 10% sodium sulfite. After filtration and drying, 142 g of high purity (95%, LC Area) of 3,4,5-tribromo-1H-pyrazole was obtained.

Example 2. Bromine/sodium hydroxide as a halogenation reagent.

34 grams of pyrazole was dissolved in water and then sodium hydroxide was added at 0 °C to obtain the corresponding pyrazole sodium salt. Next, 239.7 g of bromine was added drop-wise at 0 °C over 2 hours. The reaction temperature was controlled at 20-40 °C. After reaction, the product was precipitated as a solid, and then the reaction mixture was quenched with 10% sodium sulfite. After filtration and drying, 147 g of high purity (98%, LC Area) of 3,4,5-tribromo-1H-pyrazole was obtained.

Example 3. Potassium iodide/sodium sulfite as a dehalogenation reagent.

100 grams of 3,4,5-tribromo-1H-pyrazole, 1.1 g of KI, and 62 g of Na₂SO₃ in 300 mL DMAc were reacted at 160-180 °C for 5 hours to completed reaction. After completion of the reaction, the reaction mixture was filtered, and then DMAc was distilled off under vacuum. Next, water was added to the crude product. The reaction mixture was stirred for 10 min. The product, 3,5-dibromo-1H-pyrazole, was precipitated as a solid. After filtration and drying, 68 g of high purity (98%, LC Area) of 3,5-dibromo-1H-pyrazole was obtained.

Example 4. Coupling reaction.

22.6 grams of 3,5-dibromo-1H-pyrazole and 4.2 g of NaOH were reacted in the presence of toluene at a temperature of 150-160 °C. Water was removed by azetropic distillation under reflux temperature to yield the corresponding 3,5-dibromo-1H-pyrazole sodium salt. Then, DMAc and 15.5 g of 2,3-dichloropyridine were added and the mixture was reacted at 150-170 °C in an autoclave to produce 3-chloro-2-(3,5-dibromo-1H-pyrazol-1-yl)pyridine. After reaction, the reaction mixture was filtered. The DMAc solution containing 3-chloro-2-(3,5-dibromo-1H-pyrazol-1-yl)pyridine could be used in subsequent steps.

Example 7. Reaction in the presence of a Grignard reagent.

33.7 grams of 3-chloro-2-(3,5-dibromo-1H-pyrazol-1-yl)pyridine was dissolved in THF then iPrMgCl was added at 0 °C to yield the corresponding 3-chloro-2-(3,5-dibromo-1H-pyrazol-1-yl)pyridine magnesium salt. After 2.5 hours, 52.0 g of DMC was added drop-wise at room temperature over 6 hours. The reaction temperature was controlled at 20-40 °C. After reaction, THF and DMC were distilled off under reduced pressure, and then the reaction mixture was quenched with water. Next, toluene was added. After separation and concentration, 33 g of high purity (90%, LC Area) of methyl 3-bromo-1-(3-chloropyridin-2-yl)-1H-pyrazole-5-carboxylate was obtained.

Example 8. Hydrolysis.

33 grams of high purity (90%, LC Area) of methyl 3-bromo-1-(3-chloropyridin-2-yl)-1H-pyrazole-5-carboxylate, 12 g of 33% NaOH solution, and 90 g of toluene were charged to a flask and the mixture was heated to 80-85 °C and kept at this temperature for 1-2 hours to complete reaction. H₂SO₄ was used to adjust pH to a value in the range of about 1 to about 2 to precipitate 5-bromo-2-(3-chloro-pyridin-2-yl)-2H-pyrazole-3-carboxylic acid.

This written description uses examples to illustrate the present disclosure, including the best mode, and also to enable any person skilled in the art to practice the disclosure, including making and using any devices or systems and performing any incorporated methods. The scope of the disclosure is defined by the claims, and may include other examples that occur to those skilled in the art.

## Claims

1. A method of preparing a compound of Formula VI, wherein 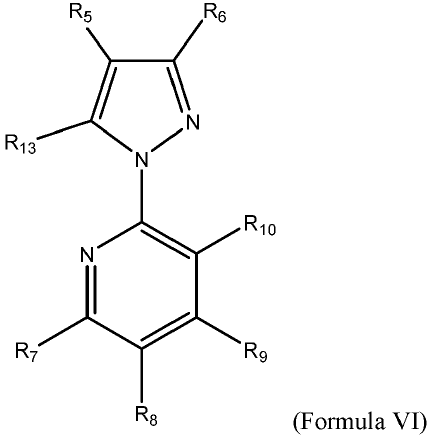
each of R₅ - R₁₀ is independently selected from hydrogen and halogen; and
R₁₃ is an organic acid, the method comprising
I) forming a mixture comprising
A) a compound of Formula V, wherein 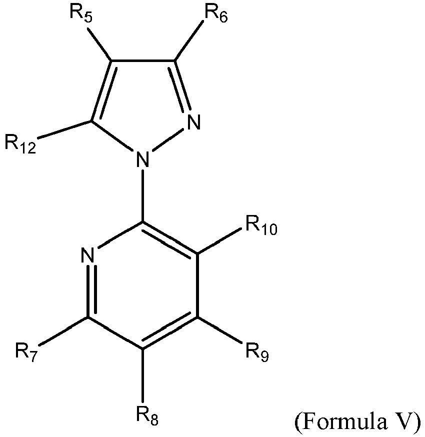
each of R₅ - R₁₀ is independently selected from hydrogen and halogen;
R₁₂ is an ester; and
wherein the compound of Formula V is prepared according to a method comprising
i) forming a mixture comprising
a) a compound of Formula III, wherein 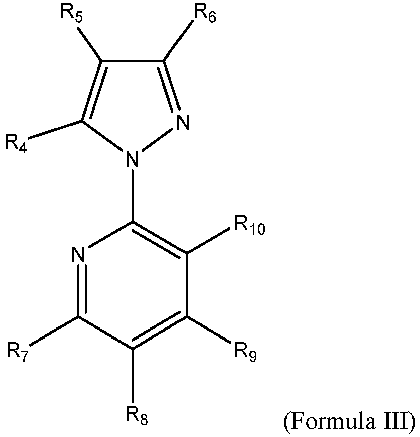 each of R₄ - R₁₀ is independently selected from hydrogen and halogen; and wherein at least one of R₄, R₅, and R₆ is hydrogen;
b) a first solvent;
c) a second solvent;
d) a compound comprising a metal; and
e) optionally an additive; and
ii) reacting the mixture; and
B) a metal hydroxide; and
II) reacting the mixture.

2. The method of claim 1, wherein the metal hydroxide is selected from alkali hydroxide, alkaline earth metal hydroxide, and combinations thereof.

3. The method of claim 1, wherein the first solvent is selected from THF, toluene, 1,4-dioxane, Me-THF, and combinations thereof.

4. The method of claim 1, wherein the second solvent is selected from dimethyl carbonate, N,N-dimethyacetamide, and combinations thereof.

5. The method of claim 1, wherein the method step ii) of reacting the mixture occurs at a reaction temperature in the range of about 0 °C to about 60 °C.

6. The method of claim 1, wherein R₄, R₅, and R₆ of Formula III are each independently hydrogen.

7. The method of claim 1, wherein the compound of Formula III is prepared by mixing a compound of Formula II with a compound of Formula IV in a solvent in the presence of an inorganic base and optionally an additive,
wherein the compound of Formula II has the following structure 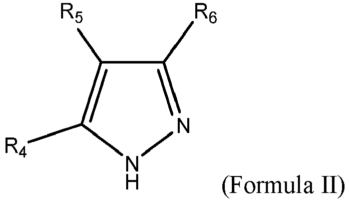
wherein each of R₄, R₅, and R₆ is independently selected from hydrogen and halogen; and
wherein at least one of R₄, R₅, and R₆ is hydrogen;
and wherein the compound of Formula IV has the following structure 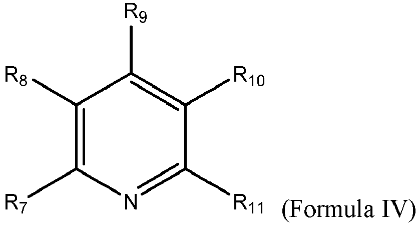 wherein each of R₇ - R₁₁ is independently selected from hydrogen and halogen.

8. The method of claim 7, wherein the compound of Formula III is prepared according to a method comprising
I) forming a mixture comprising
A) the compound of Formula II;
B) the compound of Formula IV;
C) the solvent;
D) the inorganic base; and
E) optionally the additive; and
II) reacting the mixture.

9. The method of claim 7 or claim 8, wherein:
(A) the inorganic base is selected from powder sodium hydroxide, powder potassium hydroxide, sodium carbonate, potassium carbonate, potassium phosphate, powder sodium methoxide, powder potassium t-butoxide, and combinations thereof, or
(B) the solvent is selected from toluene, N,N-dimethylformamide (DMF), N,N-dimethylacetamide (DMAc), N-methyl-2-pyrrolidone (NMP), sulfolane, diglyme, triglyme and combinations thereof, or
(C) the additive is selected from potassium iodide, a phase transfer catalyst, and combinations thereof, preferably wherein the phase transfer catalyst is selected from butyl ammonium chloride, tetra butyl ammonium bromide, aliquat-336, 18-crown-6, and combinations thereof, or
(D) the method step II) of reacting the mixture occurs at a reaction temperature in the range of about 140 °C to about 200 °C.

10. The method of claim 7 or claim 8, wherein the compound of Formula II is prepared according to a method comprising
I) forming a mixture comprising
A) a compound of Formula I, wherein 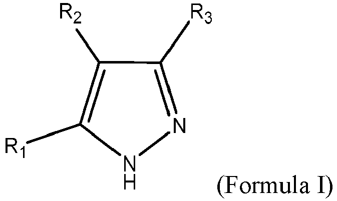 each of R₁, R₂, and R₃ is independently a halogen;
B) optionally a dehalogenation reagent;
C) a reducing agent; and
D) a solvent; and
II) reacting the mixture.

11. The method of claim 10, wherein the solvent is selected from acetic acid, water, toluene, N,N-dimethylformamide, N,N-dimethylacetamide, and combinations thereof.

12. The method of claim 10, wherein the compound of Formula I is prepared according to a method comprising
I) forming a mixture comprising
A) pyrazole or a pyrazole derivative;
B) a halogenation reagent;
C) water;
D) optionally a solvent; and
E) optionally an inorganic base; and
II) reacting the mixture.

13. The method of claim 12, wherein the halogenation reagent comprises
A) a reagent selected from hydrogen bromide, bromine, N-bromosuccinimide, 1,3-dibromo-5,5-dimethylhylhydantoin, sodium bromide, potassium bromide, and combinations thereof; and
B) optionally hydrogen peroxide.

14. A method of preparing a compound of Formula V, wherein 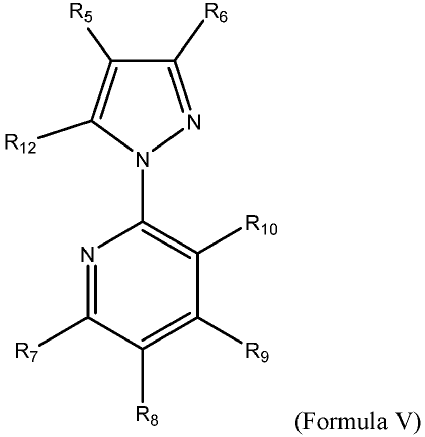
each of R₅ - R₁₀ is independently selected from hydrogen and halogen; and
R₁₂ is an ester, the method comprising
I) forming a mixture comprising
A) a compound of Formula III, wherein 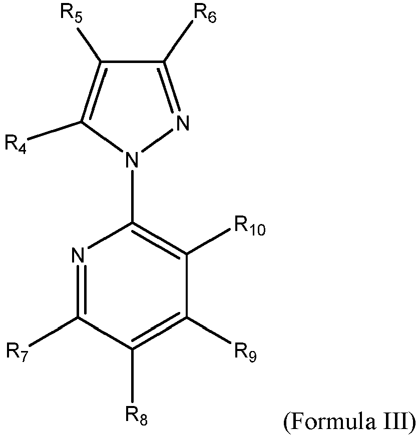
R₄ is a halogen; and
each of R₅ - R₁₀ is independently selected from hydrogen and halogen;
B) a first solvent;
C) a second solvent;
D) a compound comprising a metal; and
E) optionally an additive; and
II) reacting the mixture.

15. The method of claim 1 or claim 14, wherein the compound comprising a metal is selected from a Grignard reagent and a lithium-containing compound, preferably wherein the Grignard reagent is selected from MeMgCl, iPrMgCl, iPrMgBr, EtMgCl, and combinations thereof.

## Patentansprüche

1. Verfahren zur Herstellung einer Verbindung der Formel VI, wobei 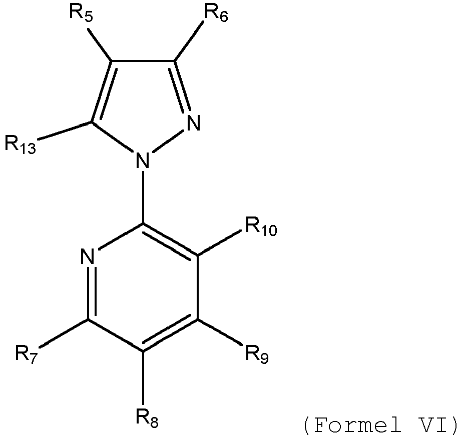
R₅ - R₁₀ jeweils unabhängig aus Wasserstoff und Halogen ausgewählt sind und
R₁₃ für eine organische Säure steht, wobei das Verfahren Folgendes umfasst:
I) Bilden einer Mischung, umfassend
A) eine Verbindung der Formel V, wobei 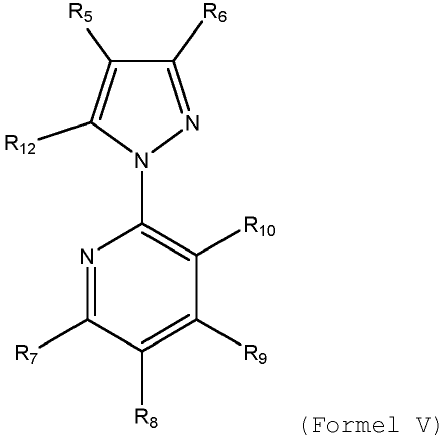
R₅ - R₁₀ jeweils unabhängig aus Wasserstoff und Halogen ausgewählt sind;
R₁₂ für einen Ester steht; und
wobei die Verbindung der Formel V gemäß einem Verfahren hergestellt wird, das Folgendes umfasst:
i) Bilden einer Mischung, umfassend
a) eine Verbindung der Formel III, wobei 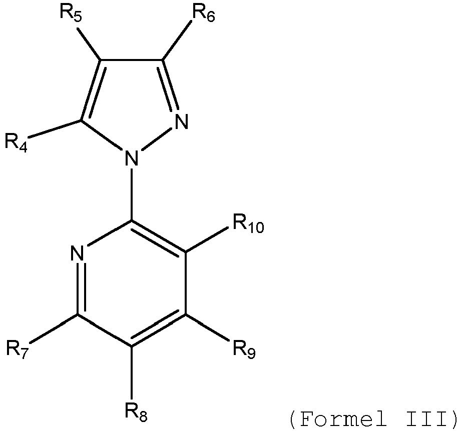 R₄ - R₁₀ jeweils unabhängig aus Wasserstoff und Halogen ausgewählt sind und wobei mindestens eines von R₄, R₅ und R₆ für Wasserstoff steht;
b) ein erstes Lösungsmittel;
c) ein zweites Lösungsmittel;
d) eine Verbindung, die ein Metall umfasst; und
e) gegebenenfalls ein Additiv; und
ii) Umsetzen der Mischung; und
B) ein Metallhydroxid; und
II) Umsetzen der Mischung.

2. Verfahren nach Anspruch 1, wobei das Metallhydroxid aus Alkalihydroxid, Erdalkalimetallhydroxid und Kombinationen davon ausgewählt wird.

3. Verfahren nach Anspruch 1, wobei das erste Lösungsmittel aus THF, Toluol, 1,4-Dioxan, Me-THF und Kombinationen davon ausgewählt wird.

4. Verfahren nach Anspruch 1, wobei das zweite Lösungsmittel aus Dimethylcarbonat, N,N-Dimethylacetamid und Kombinationen davon ausgewählt wird.

5. Verfahren nach Anspruch 1, wobei der Verfahrensschritt ii) des Umsetzens der Mischung bei einer Reaktionstemperatur im Bereich von etwa 0 °C bis etwa 60 °C erfolgt.

6. Verfahren nach Anspruch 1, wobei R₄, R₅ und R₆ von Formel III jeweils unabhängig für Wasserstoff stehen.

7. Verfahren nach Anspruch 1, wobei die Verbindung der Formel III durch Mischen einer Verbindung der Formel II mit einer Verbindung der Formel IV in einem Lösungsmittel in Gegenwart einer anorganischen Base und gegebenenfalls eines Additivs hergestellt wird,
wobei die Verbindung der Formel II die folgende Struktur aufweist: 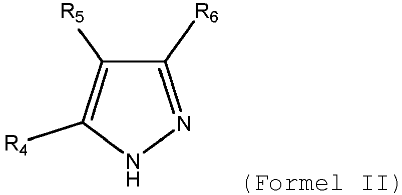
wobei R₄, R₅ und R₆ jeweils unabhängig aus Wasserstoff und Halogen ausgewählt sind und wobei mindestens eines von R₄, R₅ und R₆ für Wasserstoff steht;
und wobei die Verbindung der Formel IV die folgende Struktur aufweist: 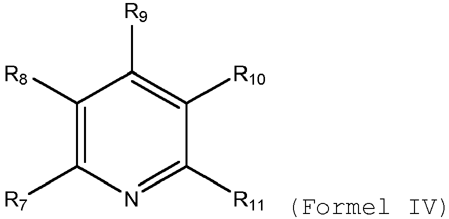
wobei R₇ - R₁₁ jeweils unabhängig aus Wasserstoff und Halogen ausgewählt sind.

8. Verfahren nach Anspruch 7, wobei die Verbindung der Formel III nach einem Verfahren hergestellt wird, das Folgendes umfasst:
I) Bilden einer Mischung, umfassend
A) die Verbindung der Formel II;
B) die Verbindung der Formel IV;
C) das Lösungsmittel;
D) die anorganische Base und
E) gegebenenfalls das Additiv; und
II) Umsetzen der Mischung.

9. Verfahren nach Anspruch 7 oder Anspruch 8, wobei:
(A) die anorganische Base aus pulverförmigem Natriumhydroxid, pulverförmigem Kaliumhydroxid, Natriumcarbonat, Kaliumcarbonat, Kaliumphosphat, pulverförmigem Natriummethoxid, pulverförmigem Kalium-t-butoxid und Kombinationen davon ausgewählt wird oder
(B) das Lösungsmittel aus Toluol, N,N-Dimethylformamid (DMF), N,N-Dimethylacetamid (DMAc), N-Methyl-2-pyrrolidon (NMP), Sulfolan, Diglyme, Triglyme und Kombinationen davon ausgewählt wird oder
(C) das Additiv aus Kaliumiodid, einem Phasentransferkatalysator und Kombinationen davon ausgewählt wird, vorzugsweise wobei der Phasentransferkatalysator aus Butylammoniumchlorid, Tetrabutylammoniumbromid, Aliquat-336, 18-Krone-6 und Kombinationen davon ausgewählt wird, oder
(D) der Verfahrensschritt II) des Umsetzens der Mischung bei einer Reaktionstemperatur im Bereich von etwa 140 °C bis etwa 200 °C erfolgt.

10. Verfahren nach Anspruch 7 oder Anspruch 8, wobei die Verbindung der Formel II nach einem Verfahren hergestellt wird, das Folgendes umfasst:
I) Bilden einer Mischung, umfassend
A) eine Verbindung der Formel I, wobei 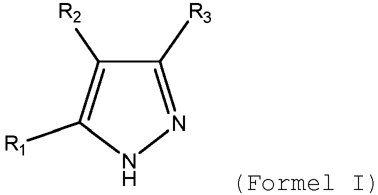 R₁, R₂ und R₃ jeweils unabhängig für ein Halogen stehen;
B) gegebenenfalls ein Dehalogenierungsreagenz;
C) ein Reduktionsmittel und
D) ein Lösungsmittel; und
II) Umsetzen der Mischung.

11. Verfahren nach Anspruch 10, wobei das Lösungsmittel aus Essigsäure, Wasser, Toluol, N,N-Dimethylformamid, N,N-Dimethylacetamid und Kombinationen davon ausgewählt wird.

12. Verfahren nach Anspruch 10, wobei die Verbindung der Formel I nach einem Verfahren hergestellt wird, das Folgendes umfasst:
I) Bilden einer Mischung, umfassend
A) Pyrazol oder ein Pyrazolderivat;
B) ein Halogenierungsreagenz;
C) Wasser;
D) gegebenenfalls ein Lösungsmittel und
E) gegebenenfalls eine organische Base; und
II) Umsetzen der Mischung.

13. Verfahren nach Anspruch 12, wobei das Halogenierungsreagenz
A) ein aus Bromwasserstoff, Brom, N-Bromsuccinimid, 1,3-Dibrom-5,5-dimethylhydantoin, Natriumbromid, Kaliumbromid und Kombinationen davon ausgewähltes Reagenz und
B) gegebenenfalls Wasserstoffperoxid umfasst.

14. Verfahren zur Herstellung einer Verbindung der Formel V, wobei 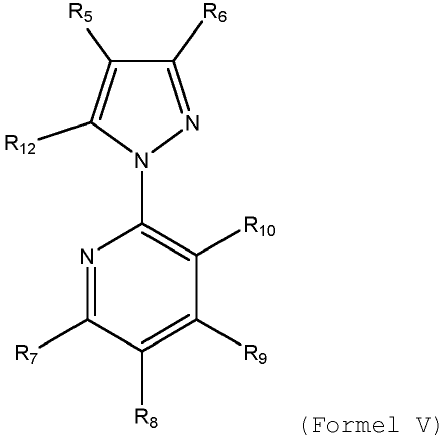
R₅ - R₁₀ jeweils unabhängig aus Wasserstoff und Halogen ausgewählt sind und
R₁₂ für einen Ester steht, wobei das Verfahren Folgendes umfasst:
I) Bilden einer Mischung, umfassend
A) eine Verbindung der Formel III, wobei 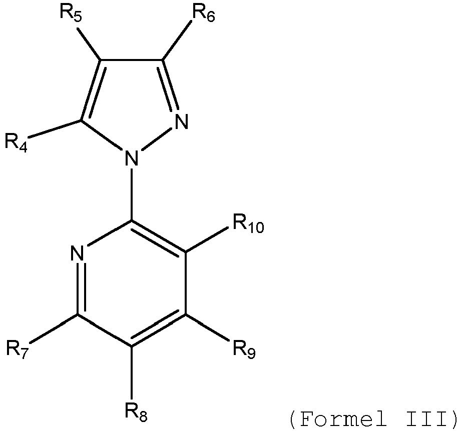
R₄ für ein Halogen steht und
R₅ - R₁₀ jeweils unabhängig aus Wasserstoff und Halogen ausgewählt sind;
B) ein erstes Lösungsmittel;
C) ein zweites Lösungsmittel;
D) eine Verbindung, die ein Metall umfasst; und
E) gegebenenfalls ein Additiv; und
II) Umsetzen der Mischung.

15. Verfahren nach Anspruch 1 oder Anspruch 14, wobei die Verbindung, die ein Metall umfasst, aus einem Grignard-Reagenz und einer lithiumhaltigen Verbindung ausgewählt wird, vorzugsweise wobei das Grignard-Reagenz aus MeMgCl, iPrMgCl, iPrMgBr, EtMgCl und Kombinationen davon ausgewählt wird.

## Revendications

1. Procédé de préparation d'un composé de formule VI, 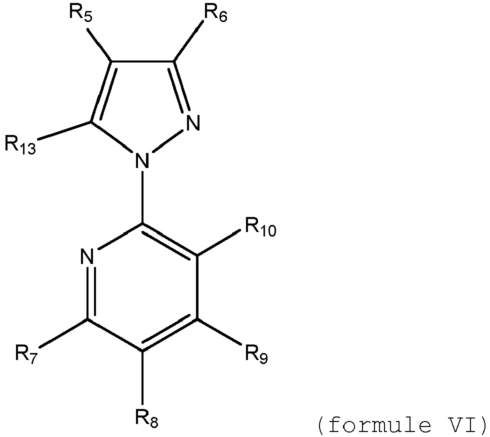
chacun parmi R₅ - R₁₀ étant indépendamment choisi parmi hydrogène et halogène ; et
R₁₃ étant un acide organique, le procédé comprenant
I) la formation d'un mélange comprenant
A) un composé de formule V, 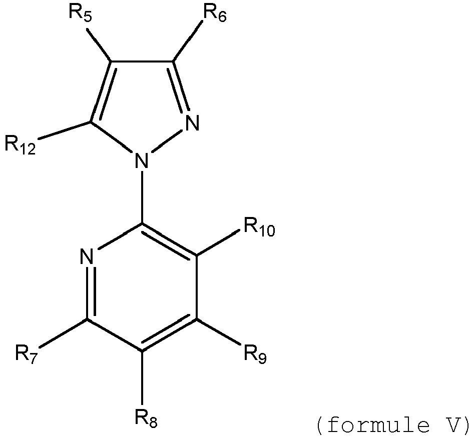
chacun parmi R₅ - R₁₀ étant indépendamment choisi parmi hydrogène et halogène ;
R₁₂ étant un ester ; et
le composé de formule V étant préparé selon un procédé comprenant
i) la formation d'un mélange comprenant
a) un composé de formule III, 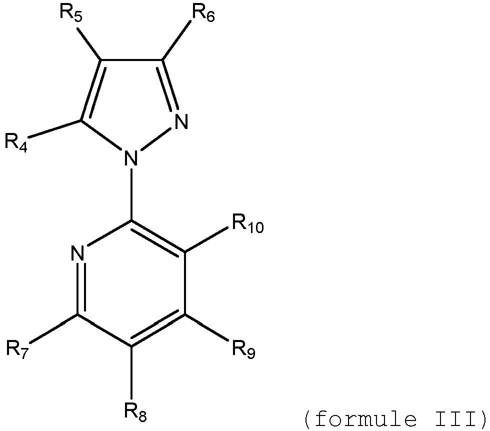 chacun parmi R₄ - R₁₀ étant indépendamment choisi parmi hydrogène et halogène ; et au moins l'un parmi R₄, R₅, et R₆ étant hydrogène ;
b) un premier solvant ;
c) un deuxième solvant ;
d) un composé comprenant un métal ; et
e) éventuellement un additif ; et
ii) la mise en réaction du mélange ; et
B) un hydroxyle métallique ; et
II) la mise en réaction du mélange.

2. Procédé selon la revendication 1, l'hydroxyde métallique étant choisi parmi un hydroxyde d'alcali, un hydroxyde de métal alcalino-terreux, et des combinaisons correspondantes.

3. Procédé selon la revendication 1, le premier solvant étant choisi parmi le THF, le toluène, le 1,4-dioxane, le Me-THF, et des combinaisons correspondantes.

4. Procédé selon la revendication 1, le deuxième solvant étant choisi parmi le carbonate de diméthyle, le N,N-diméthylacétamide, et des combinaisons correspondantes.

5. Procédé selon la revendication 1, l'étape de procédé ii) de la mise en réaction du mélange ayant lieu à une température de réaction dans la plage d'environ 0 °C à environ 60 °C.

6. Procédé selon la revendication 1, R₄, R₅, et R₆ de la formule III étant chacun indépendamment hydrogène.

7. Procédé selon la revendication 1, le composé de formule III étant préparé en mélangeant un composé de formule II avec un composé de formule IV dans un solvant en la présence d'une base inorganique et éventuellement un additif,
le composé de formule II ayant la structure suivante 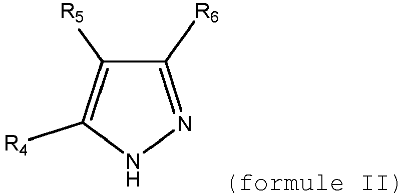
chacun parmi R₄, R₅, et R₆ étant indépendamment choisi parmi hydrogène et halogène ; et
au moins l'un parmi R₄, R₅, et R₆ étant hydrogène ;
et le composé de formule IV ayant la structure suivante 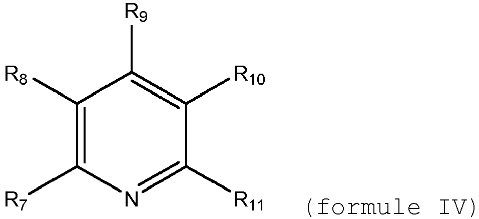
chacun parmi R₇ - R₁₁ étant indépendamment choisi parmi hydrogène et halogène.

8. Procédé selon la revendication 7, le composé de formule III étant préparé selon un procédé comprenant
I) la formation d'un mélange comprenant
A) le composé de formule II ;
B) le composé de formule IV ;
C) le solvant ;
D) la base inorganique ; et
E) éventuellement l'additif ; et
II) la mise en réaction du mélange.

9. Procédé selon la revendication 7 ou la revendication 8,
(A) la base inorganique étant choisie parmi l'hydroxyde de sodium en poudre, l'hydroxyde de potassium en poudre, le carbonate de sodium, le carbonate de potassium, le phosphate de potassium, le méthoxyde de sodium en poudre, le t-butoxyde de potassium en poudre, et des combinaisons correspondantes, ou
(B) le solvant étant choisi parmi le toluène, le N,N-diméthylformamide (DMF), le N,N-diméthylacétamide (DMAc), la N-méthyl-2-pyrrolidone (NMP), le sulfolane, le diglyme, le triglyme et des combinaisons correspondantes, ou
(C) l'additif étant choisi parmi l'iodure de potassium, un catalyseur de transfert de phases, et des combinaisons correspondantes, préférablement, le catalyseur de transfert de phases étant choisi parmi le chlorure de butylammonium, le bromure de tétrabutylammonium, l'aliquat-336, le 18-couronne-6, et des combinaisons correspondantes, ou
(D) l'étape de procédé II) de la mise en réaction du mélange ayant lieu à une température de réaction dans la plage d'environ 140 °C à environ 200 °C.

10. Procédé selon la revendication 7 ou la revendication 8, le composé de formule II étant préparé selon un procédé comprenant
I) la formation d'un mélange comprenant
A) un composé de formule I, 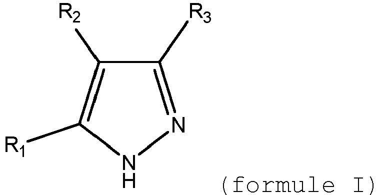 chacun parmi R₁, R₂, et R₃ étant indépendamment un halogène ;
B) éventuellement un réactif de déshalogénation ;
C) un agent réducteur ; et
D) solvant ; et
II) la mise en réaction du mélange.

11. Procédé selon la revendication 10, le solvant étant choisi parmi l'acide acétique, l'eau, le toluène, le N,N-diméthylformamide, le N,N-diméthylacétamide, et des combinaisons correspondantes.

12. Procédé selon la revendication 10, le composé de formule I étant préparé selon un procédé comprenant
I) la formation d'un mélange comprenant
A) le pyrazole ou un dérivé de pyrazole ;
B) un réactif d'halogénation ;
C) de l'eau ;
D) éventuellement un solvant ; et
E) éventuellement une base inorganique ; et
II) la mise en réaction du mélange.

13. Procédé selon la revendication 12, le réactif d'halogénation comprenant
A) un réactif choisi parmi le bromure d'hydrogène, le brome, le N-bromosuccinimide, la 1,3-dibromo-5,5-diméthylhylhydantoïne, le bromure de sodium, le bromure de potassium, et des combinaisons correspondantes ; et
B) éventuellement du peroxyde d'hydrogène.

14. Procédé de préparation d'un composé de formule V, 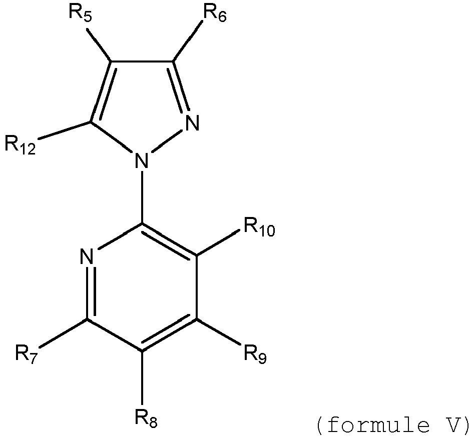
chacun parmi R₅ - R₁₀ étant indépendamment choisi parmi hydrogène et halogène ; et
R₁₂ étant un ester, le procédé comprenant
I) la formation d'un mélange comprenant
A) un composé de formule III, 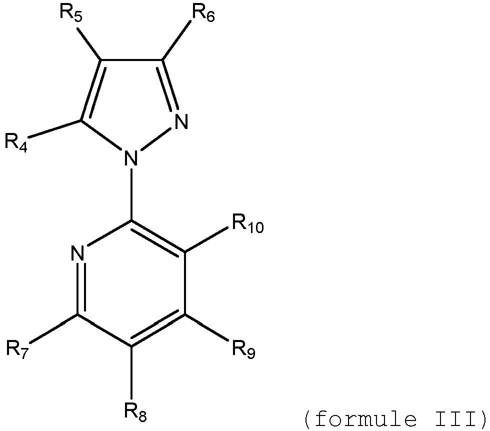
R₄ étant un halogène ; et
chacun parmi R₅ - R₁₀ étant indépendamment choisi parmi hydrogène et halogène ;
B) un premier solvant ;
C) un deuxième solvant ;
D) un composé comprenant un métal ; et
E) éventuellement un additif ; et
II) la mise en réaction du mélange.

15. Procédé selon la revendication 1 ou la revendication 14, le composé comprenant un métal étant choisi parmi un réactif de Grignard et un composé contenant du lithium, préférablement, le réactif de Grignard étant choisi parmi MeMgCl, iPrMgCl, iPrMgBr, EtMgCl, et des combinaisons correspondantes.
